# FASCICULE DE BREVET EUROPEEN

(11) **EP 3 221 628 B1**
(45) Date de publication et mention de la délivrance du brevet: **15.04.2020**
(21) Numéro de dépôt: 15804177.2
(22) Date de dépôt: 20.11.2015
(51) Int. Cl.: F16L 47/22, F16L 33/22

(54) **SYSTEME DE CONNEXION FLUIDIQUE ET PROCEDE DE FABRICATION**
FLUIDVERBINDUNGSSYSTEM UND HERSTELLUNGSVERFAHREN
FLUID CONNECTION SYSTEM AND PRODUCTION METHOD

(30) Priorité: 21.11.2014 FR 1461311
(43) Date de publication de la demande: 27.09.2017
(73) Titulaire: SARTORIUS STEDIM FMT SAS, 13400 Aubagne (FR)
(72) Inventeur: BLAKE, Florian, 13600 La Ciotat (FR); GAY, Isabelle, 13124 Peypin (FR)
(74) Mandataire: Derambure Conseil
(86) Numéro de dépôt international: PCT/FR2015/053156
(87) Numéro de publication internationale: WO 2016/079451

(56) Documents cités:
- DE-C1- 19 735 261
- DE-U1- 20 116 488
- GB-A- 808 984
- US-A- 5 566 988
- US-A1- 2005 287 326

## Description

La présente invention est relative aux systèmes de connexion fluidique pour le transfert étanche de fluides médicaux et biopharmaceutiques, et à leurs procédés de fabrication.

On connait déjà usuellement des systèmes de connexion fluidique pour le transfert étanche de fluides médicaux et biopharmaceutiques utilisant un collier permettant le maintien d'un tube sur un embout en plastique, dans la demande FR1356350. Cependant, la mise en place du collier nécessite son serrage mécanique sur le tuyau et la découpe de son extrémité, ce qui engendre des risques de pollution particulaire. Par ailleurs se pose le problème du serrage de tous les colliers selon des efforts de serrage uniformes de collier en collier, dû à la difficulté d'automatiser cette mise en œuvre. L'utilisation de tels colliers pose également le problème de la conservation d'un couple de serrage constant tout au long de la vie du produit (ce qui est rendu difficile du fait du fluage du matériau plastique constituant certains éléments du système de connexion fluidique dans le temps). Enfin, le design de ces colliers ne permet pas un serrage uniforme sur la périphérie de la connexion fluidique. Ce mode d'assemblage pénalise également la productivité en salle blanche car la mise en place du collier, son serrage et sa découpe sont réalisés manuellement et le temps technique total est particulièrement long. De plus certains matériaux constitutifs du collier peuvent constituer des sources de pollution pour des milieux très propres (salle blanche...) dans lesquels ils sont utilisés. Et finalement, malgré les précautions prises, un collier risquerait d'endommager des poches souples ou des tubes disposés dans son voisinage immédiat par une partie agressive dudit collier de serrage.

On connait également usuellement un système de connexion fluidique pour le transfert étanche de fluides médicaux et biopharmaceutiques, utilisant des bagues agrippantes. Le document EP1998096, par exemple, divulgue de telles connexions. Cependant ces systèmes de connexion présentent notamment une résistance limitée à la pression fluidique au niveau de la connexion, et un problème de dimensionnement à adapter en fonction de l'utilisation.

On connait également un système de connexion fluidique entre un connecteur et un tuyau utilisant un manchon thermorétractable, par exemple décrit dans le document DE102005024621. Ce système prévoit de coller le manchon au tuyau ou connecteur sous-jacent pour assurer la fixation et éviter l'entrée de particules extérieures au niveau de la connexion tuyau/connecteur. L'utilisation d'une colle peut être peu adaptée selon les matériaux sous-jacents, et est sensible à la température. De plus, l'utilisation d'une colle présente un risque de contamination du fluide biopharmaceutique notamment. Il y a également un risque de détérioration des composants en contact avec la colle, et de migration dans le fluide biopharmaceutique de contaminants provenant de cette détérioration.

Le document US2005/0287326 décrit un système de connexion fluidique pour le transfert stérile de liquide avec un connecteur, un tuyau et un collier serti sur le tuyau et sur le connecteur.

On cherche donc une solution alternative à l'assemblage tuyau/connecteur spécialement adapté au domaine biopharmaceutique.

A cet effet, selon l'invention, un système de connexion fluidique comprend les caractéristiques définies par la revendication 1.

Dans le cas d'un tel système de connexion, aucune colle n'est nécessaire entre les éléments à assembler, ce qui permet de réduire les risques de contamination. Avec un tel système de connexion, le serrage du tuyau et du connecteur l'un sur l'autre rattrape les tolérances de fabrication du connecteur et du tuyau, ce qui améliore l'étanchéité et la tenue mécanique. De plus, le manchon évite le risque de désolidarisation entre le connecteur et le tuyau, liée à l'augmentation de la pression intérieure du tuyau, qui risquerait d'entrainer des fuites au niveau de la région d'assemblage entre le tuyau et le connecteur.

Dans divers modes de réalisation du système de connexion selon l'invention, on peut éventuellement avoir recours en outre à l'une et/ou à l'autre des dispositions suivantes :
- la première portion embout d'assemblage mécanique du connecteur fluidique comprend au moins un cran saillant radialement vers l'extérieur, la deuxième portion d'assemblage mécanique du tuyau étant placée au-dessus dudit au moins un cran lors de l'assemblage du connecteur fluidique et de la portion souple de connexion du tuyau, le dit cran étant apte à maintenir le tuyau autour du corps rigide,
- ledit cran est adapté pour garantir un bon maintien du tuyau en traction,
- l'au moins un cran est moulé à la surface radialement extérieure du connecteur fluidique,
- la première portion embout d'assemblage mécanique du connecteur fluidique comprend une butée, ladite butée formant une surface de butée axiale pour la deuxième portion d'assemblage mécanique dans la configuration d'assemblage et une surface avant opposée à la surface de butée,

*Dans le cas d'un manchon long qui emprisonnerait la totalité de la région d'assemblage, il permettrait d'éviter le gonflement du tuyau souple à proximité de la région d'assemblage, un tel gonflement pouvant engendrer des fuites.*
- le manchon s'étend le long d'une partie du tuyau incluse strictement dans la région d'assemblage,
- les composants sont symétriques de révolution,
- le manchon est réalisé d'un seul tenant sans zone de rupture privilégiée,
- le manchon constitue l'unique moyen de rigidification du tuyau sur le connecteur fluidique,
- on place un collier de serrage serré au niveau de la région d'assemblage sur le *De plus, dans le cas éventuel de l'utilisation d'un collier de serrage au niveau de l'assemblage entre le tuyau et le connecteur, pour assurer un meilleur maintien de l'assemblage et une meilleure étanchéité, un manchon prévu autour de l'ensemble de l'assemblage et du collier permettra de protéger l'environnement contre d'éventuelles contaminations du milieu par le matériau du collier et d'éviter que la géométrie du collier ne puisse endommager d'autres éléments du milieu.*

En outre, l'invention porte sur un procédé de fabrication d'un système de connexion fluidique selon la revendication 10.

Dans un mode de réalisation du procédé selon l'invention, on peut éventuellement avoir recours en outre à la disposition suivante :
- le manchon est rétracté par application d'une température supérieure à 80°C pendant un temps supérieur à 3s au niveau du manchon.

L'absence de découpe de collier et d'arête vive permet de réduire la pollution particulaire et limite les risques de détérioration des poches par perforation par des zones saillantes.

De plus, le rétreint du manchon 1 sur le tuyau 2 pouvant être réalisé sur machine automatisée éventuellement en parallèle d'autres étapes, la productivité est améliorée.

D'autres caractéristiques et avantages de l'invention apparaîtront au cours de la description suivante d'une de ses formes de réalisation, donnée à titre d'exemple non limitatif, en regard des dessins joints.

Sur les dessins :
- La figure 1 est une représentation du système de connexion fluidique,
- La figure 2 est une représentation du montage par un connecteur entre un tuyau et une poche,
- Les figures 3 a-d sont une représentation du système de connexion fluidique lorsque le manchon couvre l'ensemble de la portion d'assemblage avant et après rétreint,
- La figure 4 présente un exemple d'installation de fabrication.
- Les figures 5 a-d sont une représentation d'un système de connexion fluidique qui ne fait pas partie de la présente invention lorsque le manchon couvre partiellement la portion d'assemblage avant et après rétreint,
- Les figures 6 a-b sont une représentation d'un système de connexion fluidique qui ne fait pas partie de la présente invention avec nervure de maintien de manchon,
- La figure 7 est une représentation du système de connexion fluidique lorsqu'un collier de serrage est préalablement utilisé pour l'assemblage,
- La figure 8 illustre un collier plastique,
- La figure 9 illustre un collier métallique,
- Les figures 10 a-b sont une représentation de variantes de la géométrie du manchon.

Sur les différentes figures, les mêmes références désignent des éléments identiques ou similaires.

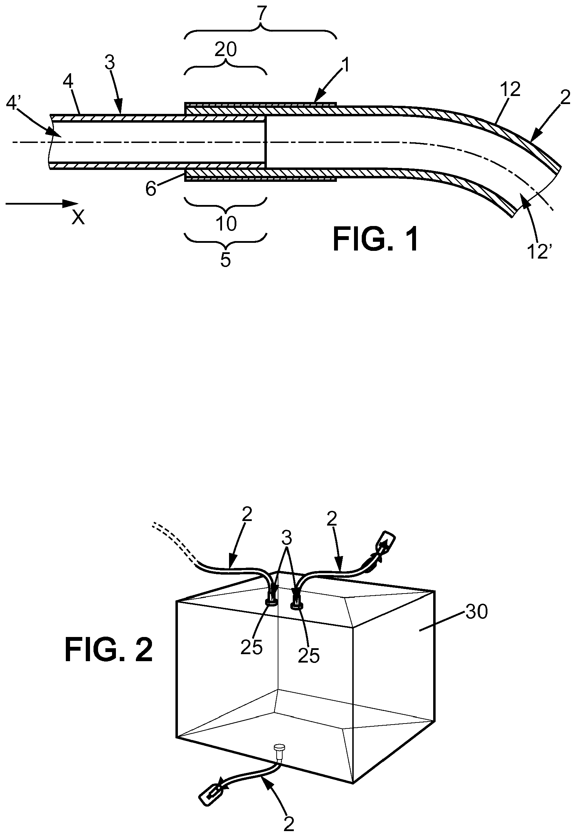

La figure 1 représente un système de connexion fluidique comprenant un manchon 1 qui emprisonne l'assemblage entre un tuyau 2 et un connecteur 3 (représentés partiellement). Le système de connexion fluidique est destiné au transfert de fluide biopharmaceutique.

Dans le contexte de l'invention, on entend par « fluide biopharmaceutique », un fluide issu de la biotechnologie - milieux de cultures, cultures cellulaires, solutions tampon, liquides de nutrition artificielle, fractions sanguines et dérivés de produits sanguins ou un fluide pharmaceutique ou plus généralement un fluide destiné à être utilisé dans le domaine médical. De tels fluides présentent préférentiellement de fortes exigences de pureté, et ne doivent pas être contaminés par des particules extérieures, qu'il s'agisse de particules des dispositifs au contact avec ces fluides pour leur contention, leur transport, ou leur traitement, ou de particules provenant de l'atmosphère environnant ces dispositifs.

En l'espèce, dans la configuration finale du système, le manchon 1 s'étend selon la direction longitudinale X d'assemblage autour du tuyau 2 et du connecteur 3.

Le manchon 1 est positionné dans une configuration initiale autour de l'assemblage entre le tuyau 2 et le connecteur 3, puis rétracté pour emprisonner l'assemblage. Le manchon s'étend sur une région de rigidification 7. La rétraction consiste en la diminution du diamètre intérieur du manchon 1, ce qui est concourant, par exemple, de la diminution du diamètre extérieur de celui-ci. On procède par exemple à une thermo-rétraction, par laquelle l'application de chaleur ou de froid au manchon 1 conduit à cette diminution. En particulier, cette application conduit à une diminution du diamètre intérieur du manchon supérieure à celle du diamètre extérieur des composants sous-jacents. Cette diminution du diamètre intérieur du manchon 1 a donc lieu alors que le tuyau 2 et le connecteur 3 restent assemblés l'un à l'autre.

Le manchon 1 est rétracté serré et libre sur le tuyau 2. Cet assemblage libre est tel que le manchon 1 thermo-rétracté reste déplaçable sur le tuyau 2 sous-jacent si on surmonte la force de friction entre le manchon 1 et le tuyau 2. En pratique, il n'est pas autrement fixé à l'assemblage que par la friction, mais n'est pas déplaçable. En effet, la force de friction entre le manchon et le tuyau 2 sera supérieure à une autre force de désassemblage du système, par exemple la force de désassemblage du tuyau 2 et du connecteur 3.

La pression permise à l'utilisation pour ce type d'assemblage de connexion après serrage du manchon peut atteindre 3 bars à l'intérieur du tuyau 2.

Comme illustré à la figure 1, le manchon 1 se présente par exemple avec une forme générale cylindrique le long de l'axe X. Le manchon 1 comprend un corps allongé creux.

Comme illustré à la figure 1, le connecteur fluidique 3 comprend au moins un corps rigide 4 le corps définissant un premier alésage 4'. Le corps du connecteur 3 comprend une première portion embout d'assemblage mécanique 10.

Comme illustré à la figure 1, le tuyau 2 comprend une portion souple de connexion 12 définissant un deuxième alésage 12' et une extrémité 6. La portion souple de connexion comprend une deuxième portion d'assemblage mécanique 20.

Le corps rigide 4 du connecteur fluidique 3 est dans un matériau biocompatible stérilisable. Le corps rigide est par exemple en plastique, polyethylène (PET), polypropylène (PP), polycarbonate, polyéthersulfone (PES) ou autre matériau adapté.

La portion souple de connexion du tuyau 2 est déformable. La portion souple est dans un matériau biocompatible stérilisable, au moins partiellement élastomère, par exemple en TPE ou en silicone. Le tuyau 2 peut être réalisé souple, ce qui permet de faciliter, par exemple, la connexion de deux conteneurs à l'aide du tuyau. De plus, la portion souple de connexion peut être déformée pour être assemblée au corps rigide 4. Le diamètre externe du tuyau 2 est par exemple au plus de 4 cm.

Comme également illustré à la figure 1, le connecteur fluidique 3 et la portion souple de connexion du tuyau 2 sont assemblés l'un à l'autre par coopération mécanique (friction) dans une région d'assemblage 5 des première 10 et deuxième 20 portions d'assemblage mécanique. Dans cette configuration d'assemblage, la portion souple 12 du tuyau 2 entoure le corps rigide 4 et est en contact serré sur celui-ci. Les premier et deuxième alésages 4', 12', sont en communication fluidique l'un avec l'autre.

L'assemblage peut se faire par exemple par insertion en force de la portion souple 12 du tuyau 2 autour du corps rigide 4, l'alésage du tuyau 2 et l'alésage du connecteur 3 étant alors placés en communication fluidique. Cette seule insertion en force peut suffire à maintenir ensemble le tuyau 2 et le connecteur 3, au moins lorsqu'aucun écoulement n'a lieu à travers le connecteur 3 et/ou en l'absence de sollicitations mécaniques importantes sur l'assemblage connecteur/tuyau.

Le connecteur 3 peut par exemple être un corps rigide creux comprenant deux extrémités, l'une des extrémités comprise dans la première portion embout d'assemblage mécanique 10 et une deuxième extrémité opposée. Les deux extrémités opposées sont en communication fluidique l'une avec l'autre. Le connecteur 3 est par exemple réalisé par moulage en une pièce.

**Le connecteur 3** est par exemple assemblé à un conteneur 30 de produit biopharmaceutique en permettant une communication fluidique avec l'intérieur de celui-ci, comme illustré à la figure 2. Le conteneur 30 est par exemple une poche souple. Le connecteur 3 est par exemple soudé à une poche par sa deuxième extrémité, par exemple par une portion 25 du corps rigide 4 autre que la première portion embout d'assemblage mécanique 10, de sorte que l'intérieur de la poche soit en communication fluidique avec l'alésage 4'.

La deuxième extrémité peut également être intégrée à un connecteur multiple de forme Y, T... pour assurer une liaison entre tuyaux, ou intégrée à un connecteur « rapide » clippé mâle ou femelle pour assurer une liaison poche avec tuyau ou tuyau avec tuyau.

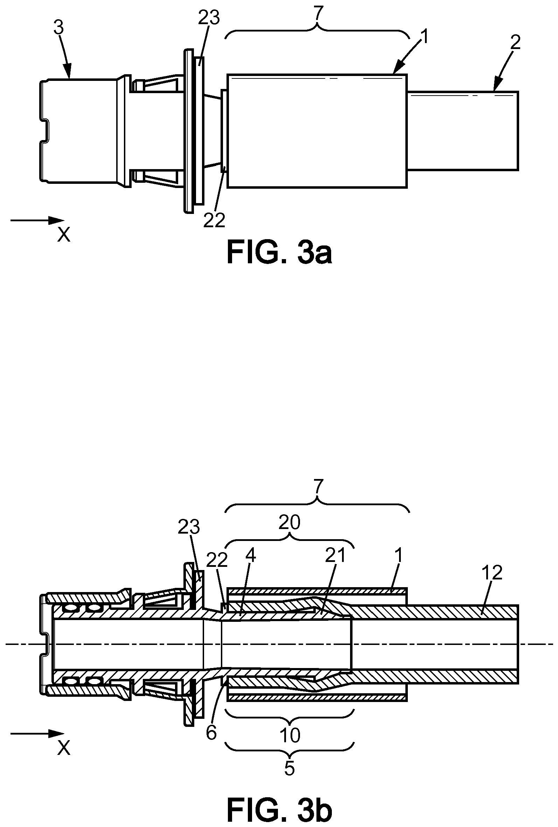

Le connecteur 3 peut également comprendre une collerette 23 (figure 3a). Ladite collerette 23 est par exemple prévue à la surface extérieure du connecteur 3. Elle s'étend radialement tout le long de la circonférence du connecteur 3. Elle comporte une face orientée vers le tuyau 2 et une face opposée selon la direction longitudinale X. Elle peut constituer un support au niveau de sa face opposée à la face orientée vers le tuyau, pour l'insertion en butée d'un autre élément sur le connecteur 3 du côté opposé à l'assemblage connecteur 3/tuyau 2 selon la direction longitudinale X.

Le manchon 1 thermo-rétractable peut être par exemple en polytéréphtalate d'éthylène (PET), polypropylène (PP), éthylène tétrafluoroéthylène (ETFE).

L'ensemble de l'assemblage décrit ci-dessus est préférentiellement à symétrie de révolution.

**Dans un premier mode de réalisation,** illustré aux figures 3a-d, les première 10 et deuxième 20 portions d'assemblage mécanique se superposent dans la région d'assemblage 5.

La première portion embout d'assemblage mécanique 10 du connecteur fluidique 3 comprend au moins un cran 21. Le cran 21 est par exemple prévu à la surface de la première portion embout d'assemblage mécanique 10 du connecteur fluidique 3, sur toute la circonférence du connecteur par exemple, ce qui permet de ne pas définir de zones à concentration de contraintes. Le cran 21 peut également être prévu sur seulement une partie de la circonférence.

Plusieurs crans, par exemple (régulièrement) espacés selon la direction axiale, peuvent être par exemple prévus sur la surface de la première portion embout d'assemblage mécanique 10.

Lors de l'assemblage du connecteur fluidique 3 et de la portion souple de connexion 12 du tuyau 2, le tuyau 2 entoure le corps rigide 4 et la deuxième portion d'assemblage mécanique 20 du tuyau 2 est placée au-dessus dudit au moins un cran 21: le tuyau 2 a alors un diamètre intérieur de maintien qui est supérieur au diamètre intérieur du tuyau 2 non monté. Cette déformation garantit un certain serrage du tuyau 2 sur le connecteur 3. Cette configuration est dite « configuration d'assemblage » du tuyau 2 et du connecteur 3.

Le cran 21 permet de maintenir le tuyau 2 lorsqu'on soumet le tuyau 2 à de la traction selon son axe longitudinal X.

Des revêtements biocompatibles disposés sur la surface radialement extérieure de la première portion embout d'assemblage mécanique 10 peuvent être utilisés pour augmenter l'adhésion de la surface du connecteur 3 au tuyau 2 en traction.

Le connecteur 3 peut par exemple également intégrer un anneau de butée 22 qui sert de butée à l'insertion du tuyau 2 sur le corps rigide 4, lors de l'assemblage du connecteur fluidique 3 et de la portion souple 12 du tuyau 2.

La butée 22 peut par exemple être prévue à la surface du corps rigide 4.

Le manchon 1 est ensuite positionné de manière lâche autour de la région d'assemblage 5 comme illustré aux figures 3a et 3b. Dans ce mode de réalisation, ledit manchon 1 s'étend dans une région de rigidification 7 prévue depuis l'extrémité 6 du tuyau 2 et au-delà de la région d'assemblage 5 sur le tuyau 2.

En variante, la région de rigidification 7 pourrait s'étendre continûment sur une partie du connecteur 3 en amont de la région d'assemblage 5 puis dans la région d'assemblage 5 puis au-delà de la région d'assemblage 5 sur le tuyau 2 selon la direction longitudinale X (non représenté).

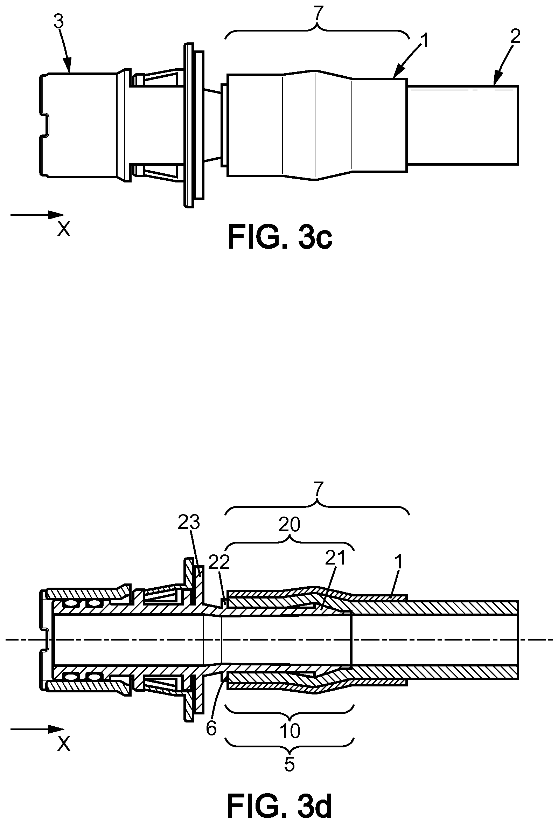

Le manchon 1 thermo-rétractable a un diamètre intérieur supérieur au diamètre extérieur du tuyau 2 en configuration d'assemblage afin de permettre ce montage. Le manchon 1 a une capacité à se rétreindre sous l'effet de la chaleur sur le tuyau afin d'assurer le maintien du tuyau sur le connecteur comme illustré aux figures 3c et 3d.

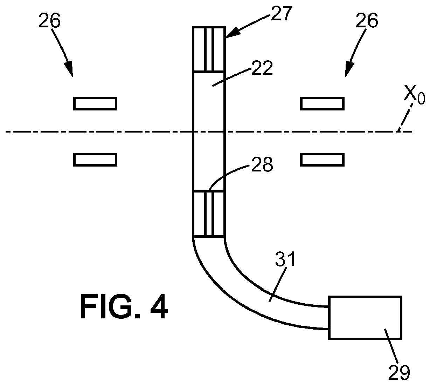

Le manchon peut être chauffé par air chaud. Le chauffage peut alors être réalisé par exemple via une ou plusieurs buse(s) d'insufflation qui projette(nt) un flux de gaz (par exemple de l'air) chaud autour du manchon 1. La figure 4 présente un exemple illustratif d'un mode de réalisation. Un système de préhension 26 est utilisé pour positionner la région d'assemblage 5 à l'endroit adapté dans l'installation de fabrication. Il s'agit par exemple d'une pince maintenant le connecteur 3 et d'une pince maintenant le tuyau 2, et disposés de manière à ce que la région d'assemblage 5 soit localisée centrée autour d'un axe X₀ d'installation d'assemblage. Un support 27 entoure l'axe X₀ et porte une pluralité de buses d'insufflation 28 disposées circonférentiellement autour de l'axe X₀. Les buses 28 sont raccordées à une source 29 de gaz chaud par l'intermédiaire d'une tuyauterie 31 adaptée. Un tel procédé peut être mis en œuvre dans des temps courts à l'échelle des temps nécessaires pour le montage de tels systèmes de connexion fluidique, par exemple pendant une durée inférieure à 1 minute, afin d'atteindre une température de rétreint au niveau du manchon supérieure à 80°C.

Le chauffage du manchon peut également être fait en approchant un élément chaud du manchon. La durée de chauffage peut aller de 3s à 30s pour des températures allant de 80°C à 350°C en fonction de la distance entre l'élément chaud et le manchon 1. On utilise par exemple un élément chaud annulaire entourant l'axe X₀.

Le procédé permet par exemple un retreint sur le tuyau 2 en quelques secondes. Le diamètre du manchon 1 serait par exemple rétreint de 50% par le présent procédé en l'absence de composants sous-jacents.

En variante encore, la chaleur peut être apportée par soudure thermique (sans contact type infra rouge). Dans un tel procédé, la source de rayonnement peut être disposée tout autour du manchon 1 à distance de celui-ci, ou être d'extension axiale limitée et déplacée axialement selon des mouvements de va-et-vient le long du manchon (selon l'axe X₀). Ceci s'applique également aux autres procédés décrits ci-dessus.

En variante, la chaleur peut être apportée par trempage dans un liquide (par exemple de l'eau) chaud.

Le manchon 1 peut également être étiré élastiquement pour en augmenter le diamètre, préalablement à son montage sur l'assemblage, pour augmenter la force de maintien. Le manchon 1 est par exemple étiré et placé sur le tuyau 2 au niveau de la région d'assemblage 5. Le tuyau 2 et le connecteur 3 sont ensuite assemblés, et le manchon 1 relâché pour enserrer élastiquement le tuyau 2. Le manchon 1 accepte l'augmentation de diamètre du tuyau 2 due à l'assemblage sur le connecteur.

Dans le cas d'un système à symétrie de révolution, le serrage du tuyau 2 sur 360° rattrape les tolérances de fabrication du connecteur 3 et du tuyau 2, ce qui améliore l'étanchéité et la tenue mécanique.

Le cran 21 permet également de maintenir en position le manchon 1 selon la direction longitudinale X.

Une fois serré, le manchon 1 applique une pression radialement et sur 360° sur le tuyau 2 afin de garantir l'étanchéité en pression de l'interface entre le connecteur fluidique 3 et le tuyau 2. Lors du passage d'un fluide biopharmaceutique, éventuellement sous pression, entre le tuyau souple et le connecteur, le manchon maintient rigidement la connexion entre le tuyau souple et le connecteur, limitant ainsi le risque de déformation du tuyau à l'interface avec le connecteur et, par conséquent, le risque de perte d'étanchéité.

En procédant de manière contrôlée à l'activation thermique du manchon 1, le tuyau 2 est donc rigidifié dans la région de rigidification 7 de manière uniforme sur la périphérie de celui-ci.

Le manchon 1 épouse la forme de l'assemblage qu'il recouvre là où il le recouvre.

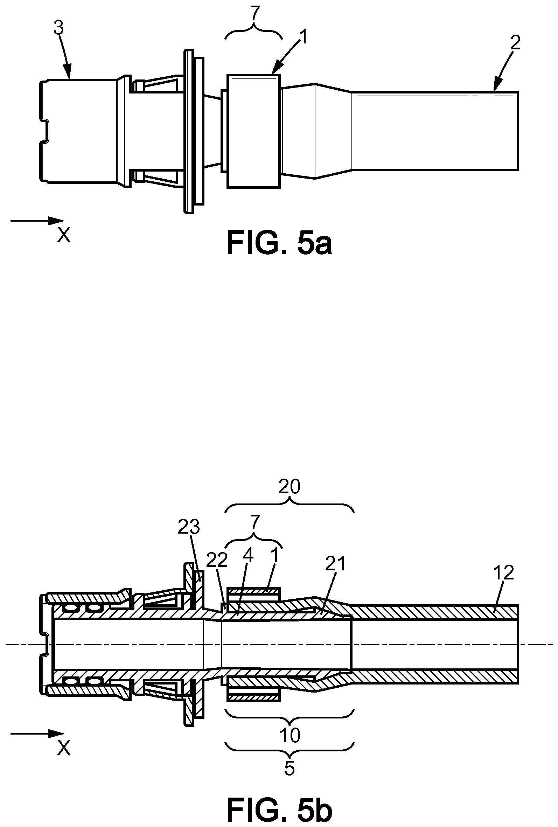

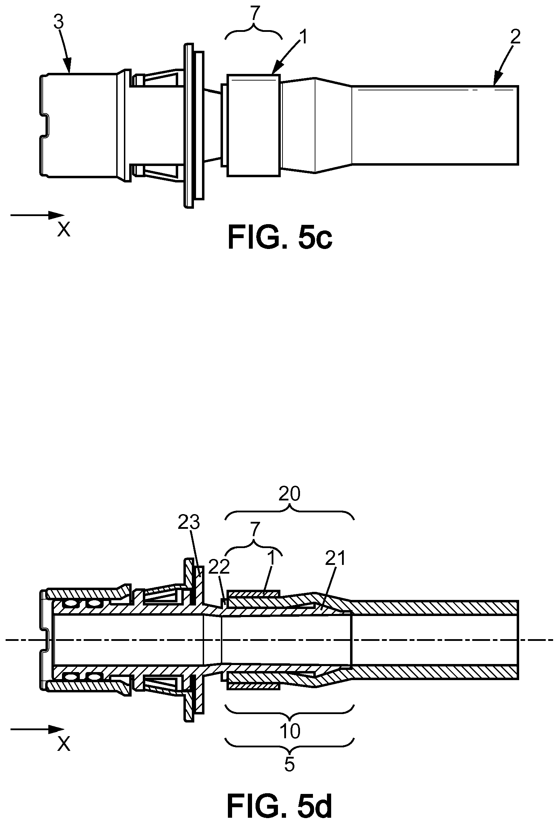

**Dans un mode de réalisation,** illustré aux figures 5a-5d, le manchon 1 est plus court que la région d'assemblage 5. Le manchon 1 s'étend dans une région de rigidification 7 prévue depuis l'extrémité du tuyau 2 et sur une partie de la région d'assemblage 5 selon la direction longitudinale X.

En variante, dans le cas où la surface de la première portion embout d'assemblage mécanique 10 comprend à la fois un cran 21 et une butée 22, le manchon 1 peut être placé axialement entre la butée 22 et le cran 21. Les modes de réalisation illustrés aux figures 5a à 5d ne sont actuellement pas protégés par le jeu de revendications de la présente application.

En variante la région de rigidification 7 pourrait s'étendre continûment sur une partie du connecteur 3 en amont de la région d'assemblage 5 puis une partie de la région d'assemblage 5 selon la direction longitudinale X (non représenté).

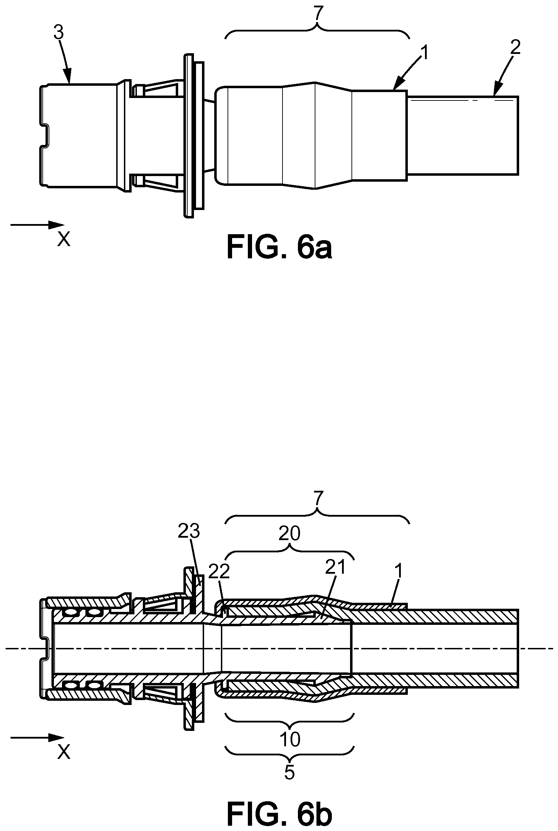

**Dans un autre mode de réalisation,** illustré aux figures 6a-6b, qui ne fait pas partie de la présente invention, le manchon 1 se retreint et s'accroche au niveau de la butée 22 de la première portion embout d'assemblage mécanique 10, le manchon englobant la butée 22 pour encapsuler au mieux l'assemblage du connecteur fluidique 3 avec la portion souple 12 de connexion du tuyau 2. Dans l'exemple présenté, le manchon 1 se rétreint sur la face de la butée 22 opposée à la face recevant le tuyau 2.

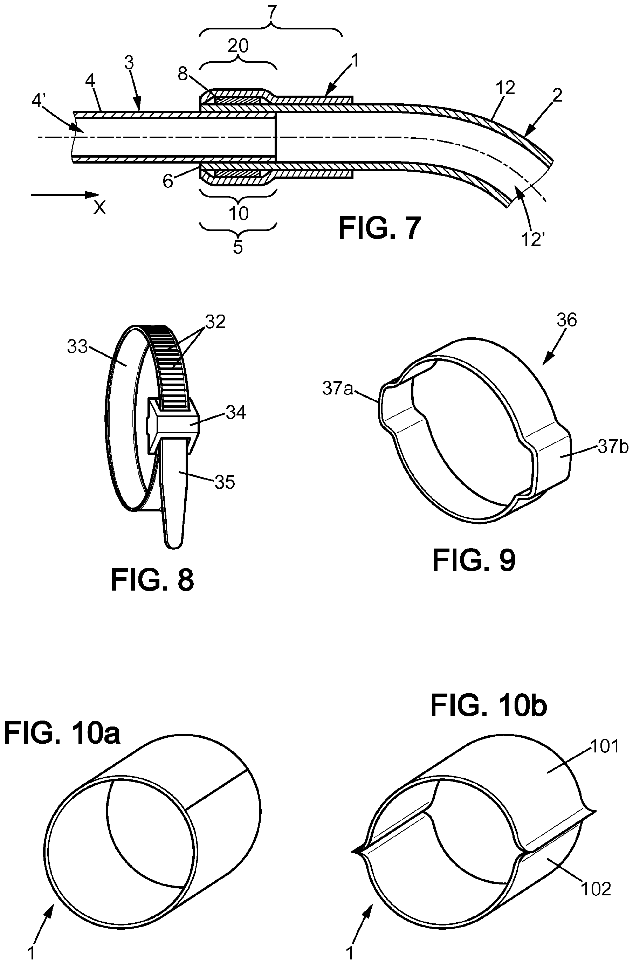

**Dans un autre mode de réalisation,** illustré à la figure 7, un collier de serrage 8 est préalablement monté sur l'assemblage décrit précédemment du connecteur 3 et du tuyau 2. Le manchon thermorétractable 1 est ensuite positionné sur l'assemblage précédent englobant l'assemblage et le collier de serrage 8, et rétreint. Le manchon 1 épouse alors la forme du collier de serrage 8 au niveau de l'assemblage.

On peut utiliser par exemple un collier en matière plastique, de type polyamide par exemple en Rilsan®. Ce type de collier plastique, illustré à la figure 8, , comprend un système de crans 32 sur une bande 33 qui coopèrent avec un crochet à verrouillage agencé dans la tête 34, de manière à ce que le serrage ne soit pas réversible. En d'autres termes, une fois que l'on a engagé la bande 33 dans la tête 34 pour former une boucle, on tire sur la bande 33 pour réduire le diamètre de la boucle et serrer le collier 8, le retour en arrière étant empêché par le crochet à verrouillage en prise dans un des crans 32 de la bande 33. Après serrage, pour éviter que la bande ne dépasse trop du diamètre de la boucle du collier, on sectionne la portion libre de bande à proximité de la tête du collier. La partie restante 35 non détachée de la bande présente souvent une arête vive qui peut s'avérer tranchante.

En alternative, on peut aussi utiliser un collier métallique tel qu'une bague à rétreindre, illustré à la figure 9, qui se présente sous la forme d'un anneau 36 préformé muni d'une ou deux oreilles 37a, 37b, faisant saillie vers l'extérieur relativement à la forme générale de l'anneau du collier, ce type de collier commercialisé par la société Oetiker®. Après insertion du collier sur le tube à maintenir, on procède au moyen d'un outil au pincement de l'oreille (ou des oreilles) du collier ce qui provoque une déformation rémanente et ainsi un rétrécissement du diamètre principal de l'anneau et par conséquent un serrage du collier sur le tube. Ce type de serrage par anneau métallique est particulièrement robuste et fiable. Toutefois, à l'endroit de l'oreille pincée par l'outil, il peut se présenter une aspérité ou une bavure qui forme une arête vive qui peut s'avérer agressive.

L'ajout du manchon 1 sur l'assemblage permet d'éviter que la partie saillante éventuelle du collier ne risque de percer par exemple des poches adjacentes lors du transport ou même de blesser l'utilisateur. Le collier est ainsi enveloppé dans le manchon 1 qui en lisse les extrémités saillantes. De plus un collier en métal notamment peut avantageusement être masqué pour éviter la contamination du milieu propre dans lequel il serait utilisé, tel que la salle blanche.

Le manchon 1 est préférentiellement à symétrie de révolution, comme illustré à la figure 10a. En variante le manchon 1 peut également se présenter sous la forme de deux demis anneaux 101 et 102 assemblés par leurs extrémités respectives comme illustré à la figure 10b. Dans le cas d'un tel manchon, les deux demis anneaux 101 et 102 sont par exemple fixés ensemble par leurs extrémités libres respectives, puis chaque jonction entre extrémités libres assemblées est aplatie de sorte que ladite jonction entre extrémités libres assemblées émerge radialement vers l'extérieur du manchon 1.

Dans une variante, le manchon 1 est monté sur le tuyau 2 avant le montage du tuyau 2 sur la première portion embout d'assemblage mécanique 10 du connecteur 3.

Dans une autre variante le tuyau 2 est monté sur la première portion embout d'assemblage mécanique 10 du connecteur 3 puis le manchon est monté sur le tuyau 2 par glissement par exemple du manchon 1 sur l'assemblage précédent.

En variante, le manchon 1 présente au moins une incision sur toute sa longueur, selon X, permettant de placer le manchon autour de l'assemblage après avoir effectué l'assemblage entre le tuyau 2 et le connecteur 3.

## Revendications

1. Système de connexion fluidique comprenant un manchon (1), un tuyau (2) et un connecteur fluidique (3),
ledit connecteur fluidique (3) comprenant au moins un corps rigide (4) dans un matériau biocompatible stérilisable, définissant un premier alésage (4'), le corps rigide (4) comprenant une première portion embout d'assemblage mécanique (10),
ledit tuyau (2) comprenant une portion souple de connexion (12) dans un matériau biocompatible stérilisable au moins partiellement élastomère, définissant un deuxième alésage (12') et une extrémité (6), la portion souple de connexion comprenant une deuxième portion d'assemblage mécanique (20),
le connecteur fluidique (3) et la portion souple de connexion (12) du tuyau (2) étant assemblés l'un à l'autre par coopération mécanique dans une région d'assemblage (5) des première portion embout (10) et deuxième portion (20) d'assemblage mécanique, dans une configuration d'assemblage dans laquelle la portion souple de connexion (12) du tuyau (2) entoure le corps rigide (4), et les premier et deuxième alésages (4', 12') sont en communication fluidique l'un avec l'autre, ledit manchon (1) étant rétracté serré sur le tuyau (2) et s'étendant dans une région de rigidification (7) prévue s'étendant sur au moins une partie de la région d'assemblage (5),
le manchon (1) s'étend le long d'une partie du tuyau (2) au-delà de la région d'assemblage (5),
**caractérisé en ce que** la région de rigidification (7) s'étend depuis l'extrémité (6) du tuyau (2) et **en ce que** le manchon (1) est thermo-rétractable et libre sur le tuyau (2) et s'étend depuis l'extrémité (6) du tuyau (2).

2. Système de connexion fluidique selon la revendication 1, pour lequel la première portion embout d'assemblage mécanique (10) du connecteur fluidique (3) comprend au moins un cran (21) saillant radialement vers l'extérieur (21), la deuxième portion d'assemblage mécanique (20) du tuyau (2) étant placée au-dessus dudit au moins un cran (21) lors de l'assemblage du connecteur fluidique (3) et de la portion souple de connexion (12) du tuyau (2), le dit cran (21) étant apte à maintenir le tuyau (2) autour du corps rigide (4).

3. Système de connexion fluidique selon la revendication 2, ledit cran (21) étant adapté pour garantir un bon maintien du tuyau en traction.

4. Système de connexion fluidique selon l'une quelconque des revendications 2 et 3 pour lequel l'au moins un cran (21) est moulé à la surface radialement extérieure du connecteur fluidique (3).

5. Système de connexion fluidique selon l'une quelconque des revendications 1 à 4 pour lequel la première portion embout d'assemblage mécanique (10) du connecteur fluidique (3) comprend une butée (22), ladite butée (22) formant une surface de butée axiale pour la deuxième portion d'assemblage mécanique (20) dans la configuration d'assemblage et une surface avant opposée à la surface de butée.

6. Système de connexion fluidique selon l'une quelconque des revendications 1 à 5, dont les composants sont symétriques de révolution.

7. Système de connexion fluidique selon l'une quelconque des revendications 1 à 6, dont le manchon (1) est réalisé d'un seul tenant sans zone de rupture privilégiée.

8. Système de connexion fluidique selon l'une quelconque des revendications 1 à 7, dont le manchon constitue l'unique moyen de rigidification du tuyau (2) sur le connecteur fluidique (3).

9. Système de connexion fluidique selon l'une quelconque des revendications 1 à 8, pour lequel on place un collier de serrage (8) serré au niveau de la région d'assemblage (5) sur le tuyau (2), avant placement du manchon (1).

10. Procédé de fabrication d'un système de connexion fluidique entre un tuyau (2) et un connecteur fluidique (3),
ledit connecteur fluidique (3) comprenant au moins un corps rigide (4) dans un matériau biocompatible stérilisable, définissant un premier alésage (4'), le corps rigide (4) comprenant un première portion embout d'assemblage mécanique (10),
ledit tuyau (2) comprenant une portion souple de connexion (12) dans un matériau biocompatible stérilisable au moins partiellement élastomère, définissant un deuxième alésage (12') et une extrémité (6), la portion souple de connexion comprenant une deuxième portion d'assemblage mécanique (20),
le procédé comprenant les deux étapes suivantes réalisées dans un ordre quelconque:
- le connecteur fluidique (3) et la portion souple de connexion (12) du tuyau (2) sont assemblés l'un à l'autre par coopération mécanique dans une région d'assemblage (5) des première (10) et deuxième (20) portions d'assemblage mécanique, dans une configuration d'assemblage dans laquelle la portion souple (12) du tuyau (2) entoure le corps rigide (4), sur une région d'assemblage (5), les premier et deuxième alésages (4', 12') étant en communication fluidique l'un avec l'autre,
- un manchon (1) est positionné lâche autour du tuyau (2), ledit manchon (1) étant en matériau thermo-rétractable, ledit manchon (1) s'étendant dans une région de rigidification (7) prévue s'étendant sur au moins une partie de la région d'assemblage (5).
le procédé comprenant ensuite l'étape suivante :
ledit manchon (1) est thermo-rétracté serré et libre sur le tuyau (2), s'étendant dans une région de rigidification (7) prévue s'étendant sur au moins une partie de la région d'assemblage (5),
la région de rigidification (7) s'étendant depuis l'extrémité (6) du tuyau (2),
le manchon (1) s'étend depuis l'extrémité (6) du tuyau (2) et le long d'une partie du tuyau (2) au-delà de la région d'assemblage (5).

11. Procédé de fabrication selon la revendication 10 pour lequel le manchon (1) est rétracté par application d'une température supérieure à 80°C pendant un temps supérieur à 3s au niveau du manchon (1).

## Patentansprüche

1. Fluidverbindungssystem, umfassend eine Hülse (1), ein Rohr (2) und ein Fluidverbindungselement (3),
wobei das Fluidverbindungselement (3) wenigstens einen starren Körper (4) aus einem sterilisierbaren, biokompatiblen Material umfasst, welches eine erste Bohrung (4') begrenzt, wobei der starre Körper (4) einen ersten mechanischen Verbindungsendabschnitt (10) umfasst,
wobei das Rohr (2) einen flexiblen Verbindungsabschnitt (12) aus einem zumindest teilweise elastomeren, sterilisierbaren, biokompatiblen Material umfasst, welches eine zweite Bohrung (12') und ein Ende (6) begrenzt, wobei der flexible Verbindungsabschnitt einen zweiten mechanischen Verbindungsabschnitt (20) umfasst,
wobei das Fluidverbindungselement (3) und der flexible Verbindungsabschnitt (12) des Rohrs (2) durch mechanisches Zusammenwirken in einem Verbindungsbereich (5) des ersten Endabschnitts (10) und des zweiten mechanischen Verbindungsabschnitts (20) miteinander verbunden sind, in einer Verbindungskonfiguration, in der der flexible Verbindungsabschnitt (12) des Rohrs (2) den starren Körper (4) umgibt und die erste und zweite Bohrung (4', 12') in Fluidverbindung miteinander stehen,
wobei die Hülse (1) dicht auf das Rohr (2) geschrumpft ist und sich in einen vorgesehenen Versteifungsbereich (7) erstreckt, der sich über wenigstens einen Teil des Verbindungsbereichs (5) erstreckt, wobei sich die Hülse (1) entlang eines Teils des Rohres (2) über den Verbindungsbereich (5) hinaus erstreckt, **dadurch gekennzeichnet, dass** sich der Versteifungsbereich (7) vom Ende (6) des Rohres (2) aus erstreckt und dass sich die Hülse (1) wärmeschrumpfbar und frei an dem Rohr (2) befindet und sich vom Ende (6) des Rohres (2) aus erstreckt.

2. Fluidverbindungssystem nach Anspruch 1, wobei der erste mechanische Verbindungsendabschnitt (10) des Fluidverbindungselements (3) wenigstens eine radial nach außen vorstehende Kerbe (21) aufweist, wobei der zweite mechanische Verbindungsabschnitt (20) des Rohrs (2) bei der Verbindung des Fluidverbindungselements (3) und des flexiblen Verbindungsabschnitts (12) des Rohrs (2) über der wenigstens einen Kerbe (21) angeordnet ist, wobei die Kerbe (21) das Rohr (2) um den starren Körper (4) herum halten kann.

3. Fluidverbindungssystem nach Anspruch 2, wobei die Kerbe (21) so angepasst ist, dass ein guter Halt des Schlauchs bei Zug gewährleistet ist.

4. Fluidverbindungssystem nach einem der Ansprüche 2 und 3, wobei wenigstens eine Kerbe (21) an der radial äußeren Fläche des Fluidverbindungselements (3) geformt ist.

5. Fluidverbindungssystem nach einem der Ansprüche 1 bis 4, wobei der mechanische Verbindungsendabschnitt (10) des Fluidverbindungselements (3) einen Anschlag (22) aufweist, wobei der Anschlag (22) eine axiale Anschlagfläche für den zweiten mechanischen Verbindungsabschnitt (20) in der Verbindungskonfiguration und eine der Anschlagfläche gegenüberliegende Vorderfläche bildet.

6. Fluidverbindungssystem nach einem der Ansprüche 1 bis 5, wobei die Komponenten rotationssymmetrisch sind.

7. Fluidverbindungssystem nach einem der Ansprüche 1 bis 6, wobei die Hülse (1) einteilig ohne Sollbruchstelle ausgeführt ist.

8. Fluidverbindungssystem nach einem der Ansprüche 1 bis 7, wobei die Hülse das einzige Versteifungsmittel des Rohrs (2) am Fluidverbindungselement (3) darstellt.

9. Fluidverbindungssystem nach einem der Ansprüche 1 bis 8, wobei vor der Platzierung der Hülse (1) eine an der Verbindungsstelle (5) geklemmte Schlauchschelle (8) am Rohr (2) platziert wird.

10. Verfahren zur Herstellung eines Fluidverbindungssystems zwischen einem Rohr (2) und einem Fluidverbindungselement (3),
wobei das Fluidverbindungselement (3) wenigstens einen starren Körper (4) aus einem sterilisierbaren, biokompatiblen Material umfasst, welches eine erste Bohrung (4') begrenzt, wobei der starre Körper (4) einen ersten mechanischen Verbindungsendabschnitt (10) umfasst,
wobei das Rohr (2) einen flexiblen Verbindungsabschnitt (12) aus einem zumindest teilweise elastomeren, sterilisierbaren, biokompatiblen Material umfasst, welches eine zweite Bohrung (12') und ein Ende (6) begrenzt, wobei der flexible Verbindungsabschnitt einen zweiten mechanischen Verbindungsabschnitt (20) umfasst, wobei das Verfahren die folgenden zwei Schritte umfasst, die in beliebiger Reihenfolge durchgeführt werden:
- das Fluidverbindungselement (3) und der flexible Verbindungsabschnitt (12) des Rohrs (2) werden durch mechanisches Zusammenwirken in einem Verbindungsbereich (5) des ersten (10) und des zweiten (20) mechanischen Verbindungsabschnitts verbunden, in einer Verbindungskonfiguration, in der der flexible Abschnitt (12) des Rohrs (2) den starren Körper (4) umgibt, in einem Verbindungsbereich (5), wobei die erste und die zweite Bohrung (4', 12') in Fluidverbindung miteinander stehen,
- eine Hülse (1) wird lose um das Rohr (2) herum positioniert, wobei die Hülse (1) aus wärmeschrumpfbarem Material besteht und wobei sich die Hülse (1) in einen vorgesehenen Versteifungsbereich (7) erstreckt, der sich über wenigstens einen Teil des Verbindungsbereichs (5) erstreckt,
wobei das Verfahren dann den folgenden Schritt umfasst:
die Hülse (1) wird dicht und frei auf das Rohr (2) wärmegeschrumpft, wobei sie sich in einem vorgesehenen Versteifungsbereich (7) erstreckt, der sich über wenigstens einen Teil des Verbindungsbereichs (5) erstreckt, wobei sich der Versteifungsbereich (7) von dem Ende (6) des Rohrs (2) aus erstreckt,
wobei sich die Hülse (1) von dem Ende (6) des Rohrs (2) und entlang eines Abschnitts des Rohrs (2) über den Verbindungsbereich (5) hinaus erstreckt.

11. Herstellungsverfahren nach Anspruch 10, wobei die Hülse (1) durch Anwendung einer Temperatur über 80°C über eine Zeit größer 3s an der Hülse (1) aufgeschrumpft wird.

## Claims

1. Fluid connection system comprising a sleeve (1), a hose (2), and a fluid connector (3),
said fluid connector (3) comprising at least one rigid body (4) made of a sterilizable biocompatible material, defining a first bore (4'), the rigid body (4) comprising a first endpiece portion for mechanical assembly (10), said hose (2) comprising a flexible connection portion (12) made of at least partially elastomeric sterilizable biocompatible material, defining a second bore (12') and an end (6), the flexible connection portion comprising a second portion for mechanical assembly (20),
the fluid connector (3) and the flexible connection portion (12) of the hose (2) being assembled to one another by mechanical engagement in an assembly region (5) of the first endpiece portion (12) and second endpiece portion (20) for mechanical assembly, in an assembly configuration in which the flexible connection portion (12) of the hose (2) surrounds the rigid body (4), and the first and second bores (4', 12') are in fluid communication with each other,
said sleeve (1) being shrunk to be tight on the hose (2) and extending over a provided stiffening region (7) extending over at least a portion of the assembly region (5),
the sleeve (1) extends along a portion of the hose (2) beyond the assembly region (5),
**characterized in that** the stiffening region (7) extends from the end (6) of the hose (2) and **in that** the sleeve (1) is thermo-shrinkable and with free movement on the hose (2) and extends from the end (6) of the hose.

2. Fluid connection system according to claim 1, wherein the first endpiece portion for mechanical assembly (10) of the fluid connector (3) comprises at least one catch (21) projecting radially outward (21), the second portion for mechanical assembly (20) of the hose (2) being positioned above said at least one catch (21) during assembly of the fluid connector (3) and the flexible connection portion (12) of the hose (2), said catch (21) being adapted to retain the hose (2) around the rigid body (4).

3. Fluid connection system according to claim 2, said catch (21) being adapted to ensure proper retention of the hose when tensile force is applied.

4. Fluid connection system according to either of claims 2 and 3, wherein the at least one catch (21) is molded in the radially outer surface of the fluid connector (3).

5. Fluid connection system according to any one of claims 1 to 4, wherein the first endpiece portion for mechanical assembly (10) of the fluid connector (3) comprises a stop (22), said stop (22) forming an axial stop surface for the second portion for mechanical assembly (20) in the assembly configuration and a front surface opposite to the stop surface.

6. Fluid connection system according to any one of claims 1 to 5, wherein the components are rotationally symmetrical.

7. Fluid connection system according to any one of claims 1 to 6, wherein the sleeve (1) is created as one piece with no predetermined breaking point.

8. Fluid connection system according to any one of claims 1 to 7, wherein the sleeve is the sole means of stiffening the hose (2) on the fluid connector (3).

9. Fluid connection system according to any one of claims 1 to 8, wherein a hose clamp (8) is tightened onto the hose (2) in the assembly region (5) before placement of the sleeve (1).

10. Method for manufacturing a fluid connection system between a hose (2) and a fluid connector (3),
said fluid connector (3) comprising at least one rigid body (4) made of a sterilizable biocompatible material, defining a first bore (4'), the rigid body (4) comprising a first endpiece portion for mechanical assembly (10), said hose (2) comprising a flexible connection portion (12) of an at least partially elastomeric sterilizable biocompatible material, defining a second bore (12') and an end (6), the flexible connection portion comprising a second portion for mechanical assembly (20),
the method comprising the following two steps carried out in any order:
- the fluid connector (3) and the flexible connection portion (12) of the hose (2) are assembled to one another by mechanical engagement in an assembly region (5) of the first (10) and second (20) portions for mechanical assembly, in an assembly configuration in which the flexible portion (12) of the hose (2) surrounds the rigid body (4), over an assembly region (5), the first and second bores (4', 12') being in fluid communication with each other,
- a sleeve (1) is positioned loosely around the hose (2), said sleeve (1) being of thermo-shrinkable material, said sleeve (1) extending over a predetermined stiffening region (7) extending over at least a portion of the assembly region (5),
the method then comprising the following step:
said sleeve (1) is thermo-shrinkable and tight but with free movement on the hose (2), extending over a predetermined stiffening region (7) extending over at least a portion of the assembly region (5),
the stiffening region (7) extending from the end (6) of the hose (2),
the sleeve (1) extending from the end (6) of the hose (2) and along a portion of the hose (2) beyond the assembly region (5).

11. Manufacturing method according to claim 10, wherein the sleeve (1) is shrunk by applying a temperature of more than 80°C for longer than 3 seconds at the sleeve (1).
